(19) Europäisches Patentamt
European Patent Office
Office européen des brevets

(11) Publication number: 0 125 892
B1

# (12) EUROPEAN PATENT SPECIFICATION

(45) Date of publication of patent specification: 08.11.89

(51) Int. Cl.$^4$: C 07 K 15/00, C 12 P 21/00, A 61 K 37/02, A 61 K 39/10

(21) Application number: 84303169.1

(22) Date of filing: 10.05.84

## (54) Islets-activating protein derivatives.

(30) Priority: 11.05.83 JP 82363/83

(43) Date of publication of application:
21.11.84 Bulletin 84/47

(45) Publication of the grant of the patent:
08.11.89 Bulletin 89/45

(84) Designated Contracting States:
DE FR GB

(56) References cited:
EP-A-0 047 802
FR-A-2 378 793
FR-A-2 393 809
JP-A-55 129 295
Huben-Weyl, Methoden der organischen Chemie,GEORG THIEME VERLAG,STUTTGART,1958 Band XI/2,pages330-331
J. P. Greenstein, M. Winitz, Chemistry of the Amino Acids, John Wiley and Sons, Inc., New York, London, Sydney, Volume 3, 1961, pages 2761-2762

The file contains technical information submitted after the application was filed and not included in this specification

(73) Proprietor: Kaken Pharmaceutical Co., Ltd.
No. 28-8, Honkomagome 2-chome Bunkyo-ku
Tokyo (JP)

(72) Inventor: Nogimori, Katsumi
7-7-6 Oe, Ohtsu-shi
Shiga-ken (JP)
Inventor: Tamura, Makoto
7-3-17 Oe, Ohtsu-shi
Shiga-ken (JP)
Inventor: Kurokawa, Shigeki
3-28-8 Seta Ohtsu-shi
Shiga-ken (JP)
Inventor: Yajima, Motoyuki
9-406 Jyutaku-Kohdan 2-3 Ohira
Ohtsu-shi Shiga-ken (JP)

(74) Representative: Woods, Geoffrey Corlett et al
J.A. KEMP & CO. 14 South Square Gray's Inn
London WC1R 5EU (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 125 892 B1

Courier Press, Leamington Spa, England.

**Description**

The present invention relates to a process for preparing a biologically active substance which comprises reacting Islets-Activating Protein having an insulin secretion promoting action with a carbonyl compound in the presence of a reducing agent and to a pharmaceutical composition containing the product of this process.

As prior art, Japanese Patent Application Laying Open Nos. 96392/78 (Application No. 10397/77) and 136592/78 (Application No. 49641/77) disclose bioligically active substances having an insulin secretion promoting action obtained by the cultivation of suitable strain(s) of the microorganism *Bordetella pertussis* (Phase I or II) in a suitable culture medium or media and a pharmaceutical composition for use in treating diabetes comprising the same. Japanese Patent Application Laying Open No. 67591/82 (Application No. 142323/80) and No. 67592/82 (Application No. 142324/80) disclose proteinic components in a biological active substance having an insulin secretion promoting action obtained by the cultivation of suitable strain(s) of the microorganism *Bordetella pertussis* (Phase I or II) in a suitable culture medium or media and furthermore, disclosure reassembled substances comprising the proteinic components.

The present invention relates to a process for preparing a modified substance from the biological active substances mentioned above, that is Islets-Activating Protein. The modified substance has a more suppressed leukocytosis-promoting effect than that of the non-modified Islets-Activating Protein, but maintains the insulin secretion promoting action.

The present invention provides a process for the preparation of a derivative of Islets-Activating Protein, which process comprises reacting Islets-Activating Protein with a carbonyl compound of the formula:

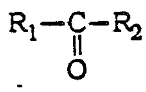

wherein $R_1$ is a hydrogen atom, methyl group or

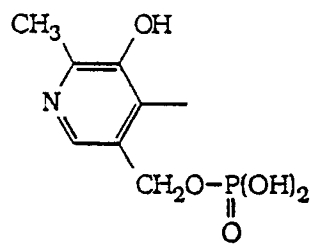

group and $R_2$ is a hydrogen atom or alkyl group of 1 to 3 carbon atoms, in the presence of a reducing agent.

The present invention also provides a pharmaceutical composition comprising as active ingredient a derivative of Islets-Activating Protein prepared by a process as defined above, together with a pharmaceutically acceptable carrier or diluent. The composition may be used for treating diabetes or as a pertussis vaccine.

The starting material is from Islets-Activating Protein (hereinafter abbreviated and referred to as IAP). IAP is obtained by the cultivation of a suitable strain of the microorganism *Bordetalla pertussis* (Phase I or II) in suitable medium or media and has the following properties.

a molecular weight of 73,000 ± 11,000 as determined by gel filtration;

a protein content as determined by Lowry's method of not less than 95% by weight;

a glucide content as determined by the phenol-$H_2SO_4$ method of less than 2% by weight;

A lipid content lower than the limit of detection;

percentage amino acid composition of the protein moiety (average ratio, μM/100 μM) of: aspartic acid, 7.5—7.9; threonine, 6.8—7.8; serine, 5.9—7.6; glutamic acid, 8.8—10.0; proline 5.5—6.4; glycine, 8.7—9.6; alanine, 9.0—10.8; cystine/2, 1.0—2.5; valine, 6.5—7.6; methionine, 2.5—3.3; isoleucine, 3.6—4.6; leucine, 7.4—8.7; tyrosine, 5.1—6.8; phenylalanine, 3.7—4.5; lysine, 3.1—4.4; histidine, 0.9—1.5; and arginine, 6.1—6.6;

disc electrophoretic pattern: acrylamide (polyacrylamide concentration, 7.5%; a 1N KOH-glacial acetic acid buffer (pH 4.3)) disc electrophoresis of said substance giving a very sharp single band on the cathode side;

hydroxyapatite column chromatographic pattern: said substance in 0.1M phosphate buffer (pH 7.0) begin adsorbed on said column and the adsorbed substance being eluted with 0.1M phosphate buffer (pH 7.0) containing 0.5M NaCl;

isoelectric point of pH 8.9 ± 0.5; and

optical rotation $[\alpha]_D^{25} = -29° \pm 5°$.

The process of the present invention introduces an alkyl group of the formula:

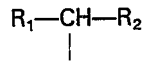

(wherein $R_1$ and $R_2$ are as defined above) or a substituted pyridylmethyl group of the formula:

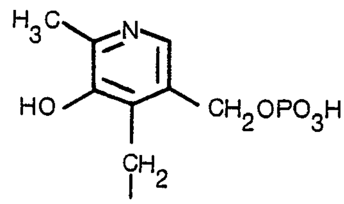

onto the free amine groups in IAP.

Suitable reducing agents used in the present invention are, for instance, sodium borohydride, sodium cyanoborohydride, or a complex of borane and a nitrogen heterocyclic compound, such as pyridine and morpholine, or an amine compound such as dimethylamine or triethylamine.

Some of the advantageous reaction conditions for modifying IAP are shown below.

1. pH of the reaction system: 6 to 10, preferably 7 to 9.
2. Reaction solvent: 0.05—0.5M, preferably 0.1M phosphate buffer solution.
3. IAP concentration: 0.01 to 1% by weight.
4. Carbonyl compound concentration: 5 to 100 mM.
5. Reducing agent concentration: 5 to 100 mM.
6. Reaction temperature: 0 to 40°C.
7. Reaction time: 5 minutes to 24 hours.

The substances produced by the present invention are such that 4 to 95%, preferably 50 to 95%, of the free amino groups per one molecule of IAP are modified by the reaction with the carbonyl compound in the presence of the reducing agent. The substances produced have a suppressed leukocytosis-promoting effect but have a hyperglycemia inhibitory action substantially equal to non-modified IAP.

The substance produced by the process of the present invention can be used in a pertussis vaccine. IAP (or N-IAP) is a pertussis infection preventive antigen but is cannot be used as such because of side effects such as its leukocytosis-promoting effect. It is therefore necessary that IAP is processed into a toxoid by a formalin or glutaraldehyde treatment. The modified IAP produced by the process of the present invention almost suppresses the side effects such as the leukocyte increasing action, and it has an effective infection preventive action without toxoid processing.

One of the purification methods, one of the modification rate determining methods, one of the electrophoresis methods and one of the activity measuring methods used in connection with the product produced according to the process of the present invention is now described in detail.

I. Purification method

2 to 3 mg of the reaction product is subjected to gel filtration with a column (1.5 × 95 cm) of Sephacryl (Trade Mark) S—200 (Pharmacia Fine Chemicals) using a 0.1M phosphate buffer solution (pH 7.0, containing 2M of urea) as the eluent. The protein content of each fraction is measured by the Lowry et al method using bovine serum albumin as standard protein.

II. Determination of modification rate

A purified sample of each of the non-modified IAP (control) and the substance produced by the process of the present invention is dissolved in distilled water (500 µg/ml). To 400 µl of this aqueous solution are added 400 µl of 0.1% trinitrobenzenesulfonic acid and 4% $NaHCO_3$ (pH 9.0), and the mixture is reacted at 37°C for 2 hours. There is then added 400 µl of 10% SDS (Sodium dodecyl sulphate), the mixture is allowed to stand at 37°C for 10 minutes and 400 µl of 1N HCl are then added. The absorbance at 335 nm is measured by a spectrometer (139 HITACHI (Trade Mark) UV—Vis spectrophotometer). The modification rate is determined by calculating the absorbance of the modified IAP derivative as a percentage of the absorbance of the non-modified IAP (given as 100%).

III. Polyacrylamide gel disc electrophoresis

The purity of the substance produced by the process of the present invention can be determined by disc electrophoresis using a polyacrylamide gel (polyacrylamide concentration, 7.5%; 1N KOH-acetate buffer (pH 4.3)). The electrophoresis is conducted by using 30 µg of the sample (in terms of protein quantity) per piece of gel at a current flow of 4 mA for 2 hours. The sample is stained with Amide Black 10B and decolorized with a 7.5% acetic acid solution.

IV. Epinephrine hyperglycemia inhibitory activity (EpI activity)

Each sample is intravenously injected into male Wistar rats (about 150 g body wieght) at a dose of 12 µg/kg, and after sample injection, epinephrine is subcutaneously injected into the rats under fast on a daily basis. The difference between the blood sugar levels before epinephrine injection and one hour after injection is compared with the difference in a control group to determine the EpI activity (%).

$\Delta G_C$: control group [(blood sugar level after epinephrine injection) — (blood sugar level before injection)]

$\Delta G_T$: sample-administered group [(blood sugar level after epinephrine injection) — (blood sugar level before injection)]

$$\text{Epl activity } (\%) = \frac{\Delta G_C - \Delta G_T}{\Delta G_C} \times 100$$

V. Leukocytosis-promoting activity ($\Delta$LP activity)

Each sample is intravenously injected into male Wister rats (about 150 g body weight) at a rate of 12 μg/kg, and the leukocyte count is measured on a daily basis. The leukocyte increment is determined from the difference in the leukocyte count between the sample-administered group and the control group. $\Delta$LP activity = (leukocyte count in sample-administered group) — (leukocyte count in control group)

VI. Preparation and purification of IAP

IAP may be prepared by the methods disclosed in Japanese Patent Application Laying Open Nos. 96392/78, 136592/78 and 67591/82.

A detailed description of the preparation of IAP and the properties of IAP are given below.

IAP having an activity of promoting the secretion of insulin is a substance which can be obtained by culturing the microorganism of *Bordetella pertussis* (Phase I or Phase II), most preferably *Bordetella pertussis* (Phase I), in a solid or liquid culture medium and, after collecting the crude product from the cultured bacterial cells and/or the culture medium, purifying the thus obtained crude product.

Collection and purification of the crude product to obtain IAP can be accomplished in general by one or more of the methods of column chromatography, dialysis, electrophoresis, gel-filtration, ion-exchange, fractional precipitation and extraction with solvent and molecular sieve. Accordingly the present invention is not restricted to any specific methods for collection and purification of the IAP.

As an example of a highly advantageous method to collect and purify the crude product to obtain IAP, a column chromatograhic process is suggested. According to this process, the supernatant of the culture medium of the bacteria is pased through a Hydroxyapatite column, a Haptoglobin Sepharose (Trade Mark) column, a Carboxymethyl Sepharose (Trade Mark) column such as a Carboxymethyl Sepharose (Trade Mark) CL—6B column (made by Pharmacia Fine Chemicals. Inc.), a Sephacryl (Trade Mark) (S—200) column, a Concanavalin Sepharose (Trade Mark) column such as a Con A-Sepharose (Trade Mark) column (made by Pharmacia Fine Chemicals. Inc.), a Biogel (P—100) (Trade Mark) column, a Biogel (Trade Mark) (P—150) column, a p-Acetoxymercurianiline Sepharose (Trade Mark) column such as a p-Acetoxymercurianiline Sepharose (Trade Mark) 6 MB column or an Anti-IAP antibody-Sepharose (Trade Mark) column such as an Anti-IAP antibody-Sepharose (Trade Mark) 4B column, to adsorb IAP. The adsorbed IAP is eluted from the column-material with a suitably selected elutant such as an aqueous Tris-hydrochloric acid solution containing KSCN, an aqueous phosphate buffer solution containing urea, or an aqueous phosphate buffer containing sodium chloride. By applying dialysis to the eluted solution, unnecessary salts are removed to obtain a fraction containing IAP.

Since IAP is necessarily also present in the cultured bacterial cells, IAP may be collected therefrom by, for example, adding sodium chloride to the aqueous suspension of the cells to leach out IAP into the aqueous solution.

The so-called precipitation method by ammonium sulfate popularly used in the art is also applicable for the preparation of IAP. In this case, solid ammonium sulfate is added to the supernatant of the culture medium of the bacteria to point approximate to saturation and the pH of the mixture is adjusted from 6 to 7 with dilute ammonia. The thus obtained precipitate is then separated and washed with water, and IAP extracted with aqueous 0.1M tis-buffer solution having a pH of 8 containing 0.5 M sodium chloride to obtain a solution of IAP.

Although *Bordetella pertussis* produces IAP as described above, its various mutants, obtained by conventional method for causing mutation, such as by modification of the components of culture medium, by exposure of the bacteria to irradiation such as ultraviolet rays or X-rays or by use of a mutagenic agent, are also useful for producing IAP.

Although the liquid-shaking culture method is preferred in view of the yield and activity of the IAP obtained, other culture methods may be used.

The mycological properties of and conditions for culturing the bacterial species belonging to the genus *Bordetella* are described in "Bergy's Manual of Determinative Bacteriology, 8th Ed.", (1974), Williams & Wilkins Co., Baltimore, Maryland, USA., "J. Exp. Med." Vol. 129, pages 523—550 (1969) and "Saikingaku Jisshu Teiyo (Handbook for Mycological Training) 3rd Ed." (1972), Maruzen Co.

The chemical and physical properties of IAP are described below.

The powder of IAP obtained by freeze-drying the aqueous dialysed and de-salted solution is non-deliquescent, is white or light brown in colour, and is soluble in water to the extent of 3 to 5 mg/ml at room temperature. It is also soluble in pyridine, 2-mercaptoethanol and an aqueous solution of sodium dodecylsulfate and of cystine. When IAP is added to an aqueous 6N hydrochloric acid solution, a white precipitate separates out. Addition of sodium sulfate, dry ice plus acetone, ethanol, trichloroacetic acid, an aqueous solution of zinc chloride, or an aqueous solution containing several other kinds of metal ions to a

aqueous solution of IAP at a cold temperature (4°C), causes turbidity in the aqueous solution and a precipitate. When IAP is added to a mixture of water and chloroform or of water and n-butanol, IAP gathers on the interface of both liquids.

When an aqueous solution of IAP is heated to 80°C or higher, the solution becomes turbid and white in colour. In addition, when IAP is dissolved in an aqueous 0.1M phosphate buffer solution having a pH of 7.0 containing 0.5 M sodium chloride and the solution is subjected to dialysis against distilled water as the external liquid, the mixed solution becomes turbid but, with the progress of dialysis, the white turbidity disappears completely.

Molecular weight of IAP:

(i) The molecular weight of IAP as determined by a gel-filtration method using a column (2.8 cm × 80 cm) of Biogel (Trade Mark) P—100 (made by Bio. Rad. Co.) equilibrated by an aqueous 0.1 M phosphate buffer solution containing 0.5 M sodium chloride is 73,000 ± 11,000.

(ii) After dissolving the active substance produced by the process of the present invention in an aqueous 0.01M potassium dihydrogen phosphate buffer solution additionally containing 0.1M sodium chloride and 2M urea, the solution is processed in "Hitachiultracentrifuge for analysis Model UCA—1" (made by Hitachi Works Co., Ltd. Japan) at 60,000 r.p.m. at 15°C to obtain the molecular weight.

The molecular weight of IAP determined by the ultracentrifugal method is 116,000 ± 7,000.

(iii) IAP is treated to slab-type SDS-polyacrylamide-disc electrophoresis (Laemmli's method) Laemmli, U.K. (1970) Nature (London) *227*, 680—685) by subjecting it to 16-hour electrophoresis using acrylamide gel with a density gradient of 10—30% under the following conditions: acrylamide gel crosslinking ratio = 37:1, concentrated gel pH = 6.8, migrating gel pH = 8.8, and electrophoresis voltage = 90 V. The resulting IAP is treated with 20% TCA (Trichloroacetic acid) for one hour and then strained with Coomassie Blue. The treated IAP is treated with 1% SDS at 100°C for 5 minutes. The molecular weight of IAP as determined by SDS-gel electrophoresis is 105,700 ± 5,000.

Component of IAP:

The content of protein in IAP determined by Lowry's method is not less than 95% by weight, and the content of glucide determined by phenol-sulfuric acid method is less than 2% by weight. The concentration of lipids in IAP is below the lower limit of detection.

The following literature are referred to for measurement of the respective components:

Protein

Lowry, O. H. , N. J. Rosebrough, A. L. Farr and R. J. Randall: "J. Biol. Chem.", *193*: 265, 1951.

Glucide

Phenol-$H_2SO_4$ method. Dobois, M., K. A. Giles, J. K. Hamilton, P. A. Rebers and F. Smith: "Anal. Chem.", *28* 350, 1956.

Lipid

Total lipid and lipid conjugate are measured according to Marsh and Weinstein method (J. B. Marsh and D. B. Weinstein: "J. Lipid Res.", *7*,574, 1966) by extracting the material before and after hydrolysis into chloroform, chloroform-methanol and heptane.

The amino acid composition of the protein component of IAP is determined according to Lowry, O.H. et al. "J. Biol. Chem.", *193* page 265 (1951), the result being as follows: (IAP is hydrolyzed in aqueous 6N hydrochloric acid at 110°C for 24 hours at a concentration of μM/100 μM).

Composition is aspartic acid, 7.5 to 7.9; theronine, 6.8 to 7.8; serine, 5.9 to 7.6; glutamic acid, 8.8 to 10.0; proline, 5.5 to 6.4; glycine 8.7 to 9.6; alanine 9.0 to 10.8; cystine/2, 1.0 to 2.5; valine, 6.5 to 7.6; methionine, 2.5 to 3.3; isoleucine 3.6 to 4.6; leucine, 7.4 to 8.7; tyrosine, 5.1 to 6.8; phenylalanine, 3.7 to 4.5; lysine, 3.1 to 4.4; histidine, 0.9 to 1.5; and arginine, 6.1 to 6.6.

Isoelectric point of IAP:

The isoelectric point of IAP determined by the method described in Wringley, C. W., "J. Chromatogr.", *36*, 362—372 (1968) is pH 8.9 ± 0.5.

Optical rotation of IAP:

The optical rotation is $[\alpha]_D^{25} = -29 \pm 5°$.

Ultraviolet absorption of IAP:

The ultraviolet absorption is $\lambda_{max}$ 277 ± 3 nm (log ε 4.79 ± 0.3)

Nuclear magnetic resonance spectrum of IAP:

The specific absorptions in the nuclear magnetic resonance spectrum are 1.2—3.5 ppm, 6.0 ppm and 6.7—8.8 ppm.

Sedimentation constant of IAP:
The sedimentation constant is $6.5 \pm 0.3S$.

Elementary Analysis of IAP (Found):
Carbon content is $46.6 \pm 6.2\%$, hydrogen content is $6.7 \pm 0.9\%$, nitrogen content is $14.4 \pm 1.2\%$, sulfur content is $1.7 \pm 0.4\%$ and oxygen content is the balance.

Biological properties of IAP:
IAP promotes the secretion of insulin at not less than 193 unit/μ g and improves the glucose tolerance in mammals. These actions are maintained for several weeks to several months after a single administration of IAP.

Acute toxicity ($LD_{50}$, i.v.) to ddY mice is about 230 μg/kg body weight.

The present invention is now explained in more detail in the following Examples.

Example 1

4 mg of IAP was dissolved in 2 ml of a 0.1M phosphate buffer solution (pH 7.0) containing 2M urea in a vessel, and 3 μl of a pyridine-borane complex dissolved in 0.1 ml of methanol (final concentration 15 mM) was added to this solution. Formaldehyde (10 μl of a 29.4 times diluted solution of 37% formalin) was then added and reacted at room temperature (20—25°C) for 2 hours. The reaction was stopped by adding 1M glycine (0.5 ml), and the reaction solution was dialyzed against distilled water by using a cellulose tube to remove the reagent and further subjected to gel filtration with a Sephacryl S—200 column to obtain 3.8 mg of a reductive methylation product of IAP (modification rate 92%) as an IAP derivative. The obtained IAP derivative showed a single band in the disc electrophoresis.

Examples 2—10

By following the procedure of Example 1 but by changing the reducing agent (sodium borohydride, sodium cyanoborohydride or pyridine-borane complex) and the kind and/or concentration of carbonyl compound, there were produced various modified IAP derivatives. The reaction conditions, modification rate and yield are shown in Table 1.

Table 1

| | IAP wt (mg) | Buffer (pH) | Reducing agent (mM) | Carbonyl compound (mM) | Reaction time (hr) | Modification rate (%) | Yield (mg) |
|---|---|---|---|---|---|---|---|
| Example 2 | 4 | 7.0 | Pyridine-borane 15 | Formaldehyde 10 | 4 | 88 | 3.9 |
| Example 3 | 4 | 8.0 | Pyridine-borane 15 | Formaldehyde 40 | 2 | 91 | 3.7 |
| Example 4 | 0.8 | 7.0 | Pyridine-borane 15 | Acetaldehyde 10 | 2 | 42 | 0.7 |
| Example 5 | 0.8 | 7.0 | Pyridine-borane 15 | Acetaldehyde 40 | 2 | 58 | 0.7 |
| Example 6 | 1 | 8.0 | Sodium cyano-borohydride 15 | Formaldehyde 20 | 1 | 89 | 0.8 |
| Example 7 | 0.8 | 7.0 | Pyridine-borane 20 | Acetone 40 | 2 | 12 | 0.7 |
| Example 8 | 0.8 | 9.0 | Pyridine-borane 15 | Acetone 40 | 2 | 22 | 0.7 |
| Example 9 | 1.0 | 8.0 | Sodium boro-hydride 50 | Pyridoxalphos-phoric acid 5 | 1 | 38 | 0.7 |
| Example 10 | 1.0 | 7.0 | Pyridine-borane 15 | Propionaldehyde 40 | 2 | 45 | 0.9 |

Experimental Test 1

The pharmacological activity, acute toxicity, and stability in an alkaline solution of the products obtained in the Examples were examined in the manner described above.

(i) Pharmacological activity:

The modified IAPs obtained in Examples 1—10 were administered to male Wistar rats as a dose of 12 μg/kg and their in vivo activities (EpI activity and ΔLP activity) one day after the administration were examined, the results being shown in Table 2. The control is non-modified IAP.

Table 2

| | EpI activity (%) | ΔLP activity (x $10^2/mm^3$) |
|---|---|---|
| Example 1 | 84.0 | 0 |
| Example 2 | 86.0 | 0 |
| Example 3 | 83.5 | 0 |
| Example 4 | 81.5 | 42.9 |
| Example 5 | 82.0 | 38.7 |
| Example 6 | 82.4 | 5.0 |
| Example 7 | 79.6 | 72.5 |
| Example 8 | 73.5 | 69.8 |
| Example 9 | 82.1 | 16.5 |
| Example 10 | 80.2 | 54.6 |
| Control | 85.0 | 140.1 |

(ii) Acute toxicity:

The acute toxicities ($LD_{50}$) of the modified IAPs obtained in Examples 1, 3, 4, 9 and 10 were examined by intravenously injecting some of the modified IAP to ddY mice (male), the results being shown in Table 3. It was found that $LD_{50}$ of the modified IAP was either equal to or higher than that of non-modified IAP. The control is non-modified IAP.

Table 3

| | $LD_{50}$ (μg/kg) |
|---|---|
| Example 1 | 250 |
| Example 3 | 232 |
| Example 4 | 215 |
| Example 9 | 240 |
| Example 10 | 236 |
| Control | 215 |

(iii) Stability:

The stabilities in an amine-containing alkaline solution of the modified IAP produced by the process of the present invention and an IAP amidino derivative disclosed in Japanese Patent Application Laid-Open No. 129295/80 (Application no. 35877/79) were examined. The results are shown in Table 4.

Table 4 shows the change with time of the EpI activity and ΔLP activity of the reductively methylated IAP obtained in Example 1 of the present invention, the acetamidinated IAP obtained in Preparation Example 1 of Japanese Patent Application Laying Open No. 129295/80 and non-modified IAP, as measured in 3.44M methylamine solution (pH 9.5). The results show that the modified IAP produced by the process of the present invention is more stable than the acetamidino-incorporated IAP in the methylamine solution.

Table 4

| | The present invention (reductively methylated IAP) | | Comparative example (acetamidinated IAP) | | Control (non-modified IAP) | |
|---|---|---|---|---|---|---|
| Treatment time (hr) | EpI activity (%) | ΔLP activity (x $10^2/mm^3$) | EpI activity (%) | ΔLP activity (x $10^2/mm^3$) | EpI activity (%) | ΔLP activity (x $10^2/mm^3$) |
| 0 | 83.5 | 0 | 81.5 | 0 | 82.0 | 144 |
| 24 | 82.0 | 0 | 80.2 | 4 | 83.0 | 141 |
| 48 | 82.5 | 0 | 76.5 | 12 | 80.5 | 138 |
| 72 | 79.8 | 0 | 75.4 | 27 | 79.8 | 139 |

Experimental Test 2

(i) Reductively methylated IAP was prepared according to the procedure of Example 1.

(ii) IAP processed into a toxoid with glutaraldehyde (hereinafter referred to a toxoid-processed IAP) to be used as a Comparative Example, was prepared according to the following procedure. To 0.1 M phosphate buffer solution (containing 2M urea, pH 7.0) containing IAP (0.5 mg/ml) adjusted to pH 9.0 by adding 1N sodium hydroxide, was added a 25% aqueous solution of glutaraldehyde such that the final concentration of glutaraldehyde was 0.05%. The mixed solution was allowed to reacted with stirring at room temperature for 22 hours. The resulting solution was then sufficiently dialyzed against a 0.1 M phosphate buffer solution (containing 2M urea, pH 7.0) to obtain the toxoid-processed IAP.

(iii) A toxoid-processed reductively methylated IAP was prepared similarly by using a reductively methylated IAP solution in place of the IAP solution.

Infection prevention test

Five physiological saline solutions containing a dead pertussis vaccine (made by KAKEN PHARMACEUTICAL CO., LTD.), toxoid-processed IAP, toxoid-processed reductively methylated IAP, IAP and reductively methylated IAP, respectively were prepared and 0.1 ml of each of these solutions was administered into the peritoneal cavities of ddY mice (male, 4 weeks old) (13 mice per one group). Two weeks after the administration, live pertussis 18—323 strain (I-phase) bacteria were inoculated into the encephalon at a rate of $4 \times 10^4$ cells/animal. The animals were observed for two weeks after the inoculation to determine the survival rate. The results are shown in Table 5. Used as the control was a group of mice which were treated similarly by having 0.1 ml of a physiological saline solution administered alone into the peritoneal cavity.

Table 5

1 bil = $10^9$ cells

| Sample | | Dose | Number of animals which survived/total number of animals tested | Survival rate (%) |
|---|---|---|---|---|
| Produced according to the present invention | Reductively methylated IAP | 0.04 µg/animal | 7/13 | 54 |
| | | 0.2 | 6/13 | 46 |
| | | 1 | 9/13 | 69 |
| | Toxoid-processed reductively methylated IAP | 0.2 µg/animal | 7/13 | 54 |
| | | 1 | 8/13 | 62 |
| | | 5 | 11/13 | 85 |
| Comparative Example | Dead pertussis vaccine | 0.4 bil/animal | 9/13 | 69 |
| | Toxoid-processed IAP | 0.2 µg/animal | 7/13 | 54 |
| | | 1 | 8/13 | 62 |
| | | 5 | 10/13 | 77 |
| | IAP | 0.04 µg/animal | 6/13 | 46 |
| | | 0.2 | 8/13 | 62 |
| | | 1 | 3/13 | 23 |
| Control | | 0 | 2/13 | 15 |

As seen from the above table, the reductively methylated IAP showed an effective infection preventive action without toxoid processing with glutaraldehyde. When the reductively methylated IAP was subjected to toxoid processing, it showed a preventive action almost equal to that of the toxoid-processed non-modified IAP.

The active substances produced by the process of the present invention are very useful in the treatment and prevention of diabetes or in pertussis vaccines. The effective dose for human applications varies depending on specific activity of the active substances. Usually, for promoting insulin promoting secretory activity, they are administered in an amount of from 10 ug/kg (body weight) to several ten µg/kg (body weight).

Intravenous injection is the most effective route of administration, but there may be employed other modes of administration such as intraperitoneal, intramuscular or subcutaneous injection, direct administration into the digestive organs, or oral, intrarectal, sublingual, nasal mucosal, intra-arterial, intralymphangial or intratracheal administration.

The composition to be administered may be in the form of an injectable composition, suppository, enteric or gastric coatings, sublingual tablet or inhalant. A simple example of an injectable compositions is a 1 ml mixture of 10,000 units of insulin secretory active substance, 9 mg NaCl and sterile distilled water.

The definition of the activity units used in the present invention is described in Japanese Patent Application Laying Open No. 96392/72, which corresponds to GB—A—1,569,046.

It will be apparent to those skilled in the art that other additives which do not affect the activity of the active substance may be mixed with the medicaments.

**Claims**

1. A process for the preparation of a derivative of Islets-Activating Protein, which process comprises reacting Islets-Activating Protein with a carbonyl compound of the formula:

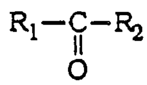

wherein $R_1$ is a hydrogen atom, methyl group or

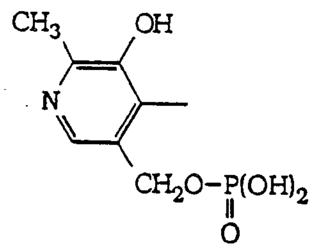

group and $R_2$ is a hydrogen atom or alkyl group of 1 to 3 carbon atoms, in the presence of a reducing agent.

2. A process according to claim 1, in which from 50 to 95% of the free amino groups of Islets-Activating Protein are modified by the reaction with the carbonyl compound.

3. A process according to any one of the preceding claims, in which the reducing agent is sodium borohydride, sodium cyanoborohydride, pyridine borane, morpholine borane or a complex of an amine and borane.

4. A process according to claim 3, in which the complex of amine and borane is dimethylamine borane or triethylamine borane.

5. A process according to any one of the preceding claims, in which reaction is effected at from 0 to 40°C for from 5 minutes to 24 hours.

6. A pharmaceutical composition comprising as active ingredient a derivative if Islets-Activating Protein prepared by a process as claimed in any one of the preceding claims, together with a pharmaceutically acceptable carrier or diluent.

7. A composition according to claim 6 in unit dosage form for use in treating diabetes.

8. A composition according to claim 6 in unit dosage form for use as a pertussis vaccine.

**Patentansprüche**

1. Verfahren zur Herstellung eines Derivats des Inseln aktivierenden Proteins, welches darin besteht, ein Inseln aktivierendes Protein mit einer Carbonylverbindung der Formel

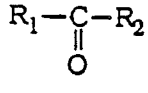

worin $R_1$ ein Wasserstoffatom, eine Methylgruppe oder ein Gruppe der Formel

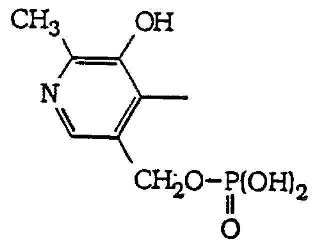

und $R_2$ ein Wasserstoffatom oder eine Alkylgruppe mit 1 bis 3 Kohlenstoffatomen bedeuten, in Gegenwart eines Reduktionsmittels umsetzt.

2. Verfahren nach Anspruch 1, worin 50 bis 95% de freien Aminogruppen des Inseln aktivierenden Proteins durch die Umsetzung mit der Carbonylverbindung modifiziert werden.

3. Verfahren nach einem der vorhergehenden Ansprüche, worin als Reduktionsmittel Natriumborhydrid, Natriumcyanoborhydrid, Pyridin-Boran, Morpholin-Boran oder ein Komplex aus einem Amin und Boran verwendet wird.

4. Verfahren nach Anspruch 3, worin als Komplex aus dem Amin und Boran Dimethylamin-boran oder Triethylamin-Boran verwendet wird.

5. Verfahren nach einem der vorhergehenden Ansprüche, worin die Reaktion bei 0 bis 40°C während 5 Minuten bis 24 Stunden durchgeführt wird.

6. Pharmazeutische Zubereitung enthaltend als Wirkstoff ein Derivat des Inseln aktivierenden Proteins hergestellt nach einem Verfahren gemäß einem der vorhergehenden Ansprüche, in Kombination mit einem pharmazeutisch annehmbaren Trägermaterial oder Verdünnungsmittel.

7. Zubereitung nach Anspruch 6 in Dosiseinheitsform zur Behandlung von Diabetes.

8. Zubereitung nach Anspruch 6 in Dosiseinheitsform zur Verwendung als Keuchhusten-Impfstoff.

**Revendications**

1. Procédé pour la préparation d'un dérivé de Protéine Activant les Ilots, lequel procédé comprend la réaction d'une Protéine Activant les Ilots avec un composé carbonylé répondant à la formule:

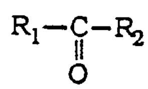

dans laquelle $R_1$ est un atome d'hydrogène, un groupe méthyle ou un groupe

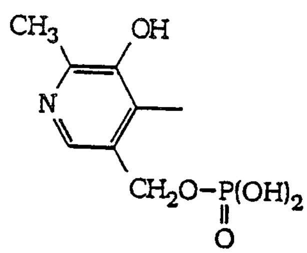

et $R_2$ est un atome d'hydrogène ou un groupe alkyle en $C_1$ à $C_3$, en présence d'un agent réducteur.

2. Procédé suivant la revendication 1, dans lequel de 50 à 95% des groupes amino libres de la Protéine Activant les Ilots sont modifiés par la réaction avec le composé carbonylé.

3. Procédé suivant l'une quelconque des revendications précédentes, dans lequel l'agent réducteur est le borohydrure de sodium, le cyanoborohydrure de sodium, la pyridine borane, la morpholine borane ou un complexe d'une amine et du borane.

4. Procédé suivant la revendication 3, dans lequel le complexe d'amine et de borane est le diméthylamine borane oü le triéthylamine borane.

5. Procédé suivant l'une quelconque des revendications précédentes, dans lequel la réaction est effectuée à une température de 0 à 40°C pendant une durée de cinq minutes à vingt-quatre heures.

6. Composition pharmaceutique comprenant comme ingrédient actif un dérivé de la Protéine Activant les Ilots préparé par un procédé tel que revendiqué dans l'une quelconque des revendications précédentes, avec un support ou diluant pharmaceutiquement acceptable.

7. Composition suivant la revendication 6 sous forme de dose unitaire pour l'utilisation dans le traitement du diabète.

8. Composition suivant la revendication 6 sous forme de dose unitaire pour l'utilisation comme vaccin anticoquelucheux.